(19) Europäisches Patentamt
European Patent Office
Office européen des brevets

(11) Veröffentlichungsnummer: **0 101 574**
**B1**

(12) # EUROPÄISCHE PATENTSCHRIFT

(45) Veröffentlichungstag der Patentschrift: **04.02.87**

(51) Int. Cl.4: **C 07 D 471/04**, A 61 K 31/435 // (C07D471/04, 221:00, 209:00)

(21) Anmeldenummer: **83107370.5**

(22) Anmeldetag: **27.07.83**

(54) **2-Substituierte 1-Aminoalkyl-1,2,3,4-tetrahydro-beta-carboline, ihre Herstellung und Verwendung als Arzneimittel.**

(30) Priorität: **05.08.82 DE 3229214**
**17.03.83 DE 3309596**

(43) Veröffentlichungstag der Anmeldung: **29.02.84 Patentblatt 84/9**

(45) Bekanntmachung des Hinweises auf die Patenterteilung: **04.02.87 Patentblatt 87/6**

(84) Benannte Vertragsstaaten: **AT BE CH DE FR GB IT LI NL SE**

(56) Entgegenhaltungen:
**EP-A-0 021 857**

**PATENTS ABSTRACTS OF JAPAN, Band 6, Nr. 98 (C-106) 976, 8. Juni 1982**
**CHEMICAL COMMUNICATIONS, 1968, Seite 410, London, GB S.R. JOHNS u.a.: "Elaeocarpidine, a new indole alkaloid from Elaeocarpus archboldianus A.C.Sm."**

(73) Patentinhaber: **BASF Aktiengesellschaft, Carl-Bosch- Strasse 38, D-6700 Ludwigshafen (DE)**

(72) Erfinder: **Steiner, Gerd, Dr., Oberer Waldweg 1, D-6719 Kirchheim (DE)**
Erfinder: **Mueller, Claus D., Dr., Odenwaldring 84, D-6806 Viernheim (DE)**
Erfinder: **Lenke, Dieter, Dr., Kekuleplatz 1, D-6700 Ludwigshafen (DE)**

**EP 0 101 574 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft 2-substituierte 1-(Aminoalkyl)-1,2,3,4-tetra-hydro-β-carboline, Verfahren zu deren Herstellung sowie diese enthaltende therapeutische Mittel und deren Verwendung bei der Bekämpfung von Krankheiten.

Aus der JP-OS 57-28087 (vgl. Patent Abstracts of Japan 98, 976 (1982)) sind bereits 1-(3'-Aminopropyl)-tetrahydro-β-carbolin-Verbindungen bekannt, die als Zwischenprodukten zur Herstellung von Wirkstoffen und Indolalkaloiden dienen. Weiter ist aus Chem. Comm. 1968, 410 N-Acetyl-dihydrodacocarpidin bekannt, welches aus Elaeocarpidin durch katalytische Hydrierung und Acetylierung entsteht.

Es wurde gefunden, daß 2-substituierte 1-(Aminoalkyl)-1,2,3,4-tetra-hydro-β-carboline der Formel I

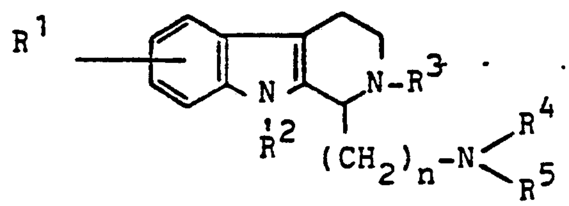

I,

in der

n eine ganze Zahl von 2 bis 5,

$R^1$ ein Wasserstoff- oder Halogenatom oder einen Trifluormethyl-, $C_1$-$C_4$-alkyl- oder $C_1$-$C_4$-Alkoxyrest,

$R^2$ ein Wasserstoffaton oder einen $C_1$-$C_3$-Alkylrest,

$R^3$ einen $C_1$-$C_3$-Acylrest,

$R^4$ einen Alkylrest mit bis zu 4 C-Atomen,

$R^5$ ein Wasserstoffatom oder einen Alkylrest mit bis zu 4 C-Atomen oder

$R^4$ und $R^5$ zusammen mit dem N-atom einen Piperidinring, der durch eine oder mehrere $C_1$-$C_3$-Alkylgruppen substituiert sein kann, bedeuten, sowie deren Salze mtt physiologisch verträglichen Säuren wertvolle pharmakologische Eigenschaften besitzen.

Der Rest $R^1$ ist vorzugsweise Wasserstoff. Methyl oder Chlor, $R^2$ vorzugsweise Wasserstoff, Methyl oder Ethyl und $R^3$ vorzugsweise $C_2$-$C_3$-Acyl, insbesondere Acetyl, n ist vorzugsweise 3.

Für den Rest $-NR^4R^5$ seien insbesondere Dialkylamino und Monoalkylamino genannt - wobei Dialkylamino bevorzugt ist -, sowie weiter der Piperidinring.

Die folgenden Verbindungen sind als besonders wirksam zu nennen:

1 [3'-(Di-2-methylpropyl)-aminopropyl]-2-acetyl-9-methyl-1,2,3,4-tetra-hydro-β-carbolin

1-(3'-Dipropylaminopropyl)-2-acetyl-9-methyl-1,2,3,4-tetrahydro-β-carbolin

1-(3'-Isopropyl-propylaminopropyl)-2-acetyl-9-methyl-1,2,3,4-tetrahydro-β-carbolin.

Weiter seien genannt:

1-[3'-Diisopropylaminopropyl]-2-acetyl-9-methyl-1,2,3,4-tetrahydro-β-carbolin

1-[3'-(Di-2-butyl)-aminopropyl]-2-acetyl-9-methyl-1,2,3,4-tetrahydro-β-carbolin

1-[3'-(t-Butyl)-aminopropyl]-2-acetyl-9-methyl-1,2,3,4-tetrahydro-β-carbolin

1-[3'-(Isopropyl-2-methylpropyl)-aminopropyl]-2-acetyl-9-methyl-1,2,3,4-tetrahydro-β-carbolin

1-3'-(Di-n-butyl-aminopropyl)-2-acetyl-9-methyl-1,2,3,4-tetrahydro-β-carbolin

1-[3'-(Di-2-butyl)-aminopropyl]-2-acetyl-1,2,3,4-tetrahydro-carbolin

1-[3'-(Di-n-butyl)-aminopropyl]-2-acetyl-1,2,3,4-tetrahydro-carbolin

Die erfindungsgemäßen Verbindungen der Formel 1 werden hergestellt, indem man eine Verbindung der Formel II

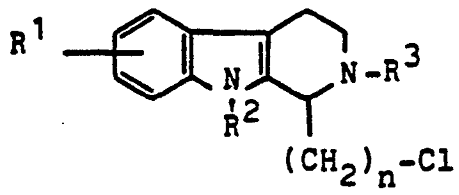

II

in der n, $R^1$, $R^2$ und $R^3$ die angegebenen Bedeutungen haben, mit einem Amin der Formel
$HNR^4R^5$,
worin $R^4$ und $R^5$ die angegebenen Bedeutungen haben, umsetzt und die so erhaltenen Verbindungen gegebenenfalls in ihre Salze mit physiologisch verträglichen Säuren überführt.

Die Umsetzung erfolgt zweckmäßig in Gegenwart eines Moläquivalents eines tertiären Amins wie z.B. Triethylamin in einem inerten Lösungsmittel, wie einem cyclischen gesättigten Ether, insbesondere Tetrahydrofuran oder Dioxan, oder einem polaren aprotischen Lösungsmittel, vorzugsweise Dimethylformamid, bei Temperaturen von 0 bis 150°C und ist im allgemeinen innerhalb von 3 bis 10 Stunden beendet. Es kann auch in Gegenwart einer überschüssigen Menge des verwendeten Aminoalkohols III, der gleichzeitig als Lösungsmittel und als säurebindendes Mittel dienen kann, gearbeitet werden.

Die freien Verbindungen der Formel I werden in der Mehrzahl der Fälle in Form von Kristallen erhalten und können durch Umkristallisation aus den üblichen organischen Lösungsmitteln, bevorzugt aus einem niederen Alkohol, wie Ethanol oder einem niedrigen Ester, vorzugsweise Essigsäureethylester, umkristallisiert oder durch Säulenchromatographie gereinigt werden.

Gegebenenfalls werden die erhaltenen erfindungsgemäßen Verbindungen in das Säureadditionssalz einer physiologisch verträglichen Säure überführt. Als übliche physiologisch verträglich organische oder anorganische Säuren kommen beispielsweise in Betracht: Salzsäure, Bromwasserstoffsäure, Phosphorsäure oder Schwefelsäure sowie Oxalsäure, Maleinsäure, Fumarsäure, Milchsäure, Weinsäure, Äpfelsäure, Zitronensäure, Salicylsäure, Adipinsäure oder Benzoesäure. Weitere Säuren können beispielsweise dem J. Pharm. Sciences 66, 1 (1977) entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol oder einem niederen Keton wie Aceton, Methylethylketon oder Methylisobutylketon oder einem Ether wie Diethylether, Tetrahydrofuran oder Dioxan, erhalten. Zur besseren Kristallabscheidung können auch Mischungen der genannten Lösungsmittel verwendet werden.

Die Ausgangsverbindungen der Formel II werden beispielsweise erhalten, indem man ein β-Carbolin-Derivat der Formel III

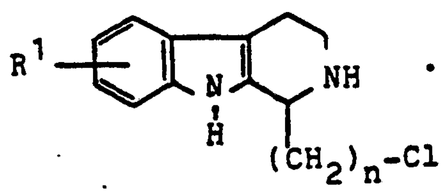

· HCl

III,

in der
n und $R^1$ die angegebene Bedeutung hat, mit einem Acylierungsmittel $R^3$-Cl, worin $R^3$ die angegebene Bedeutung hat, unter Abfangbedingungen in Gegenwart einer Base vorzugsweise Pyridin, in einem inerten Lösungsmittel, vorzugsweise Dimethylformamid, unter Kühlung vorzugsweise bei 0 bis 15°C umsetzt.

Falls $R^2$ in dem gewünschten Endprodukt einen Alkylrest darstellt, wird dieser durch Umsetzen der N-2-Acylverbindung mit Alkylchlorid oder Dimethylsulfat in einem inerten Lösungsmittel erhalten.

Die Verbindungen III lassen sich durch Umsetzen der entsprechenden Tryptamin-Derivate mit einem ω-Halogenaldehyd in schwach saurem Medium bei 50 bis 100°C herstellen.

Die erfindungsgemäßen Verbindungen und deren physiologisch verträglichen Säureadditionssalze weisen wertvolle pharmakologische Eigenschaften auf. Sie sind stark antiarrhythmisch wirksam und eignen sich daher inbesondere zur Pharmakotherapie von Herzrhythmusstörungen.

Zur Bestimmung der antiarrhythmischen Wirksamkeit werden die Substanzen männlicher Ratten (Stamm: Sprague-Dawley, Gewicht 200 bis 250 g) oral appliziert. 45 min später werden die Tiere mit Thiobutabartial-Natrium (100 mg/kg i.p.) narkotisiert. Als arrhythmogene Substanz dient Aconitin, das 60 min nach Substanzapplikation i.v. infundiert wird (Dosierungsgeschwindigkeit: 0,005 mg/kg . min). Bei nicht behandelten Tieren (N = 52) treten nach 2,74 ± 0,07 min im EKG Arrhythmien auf, deren Eintritt durch Antiarrhythmica dosisabhängig verzögert werden kann.

Zwischen den Dosenlogarithmen (mg/kg) der Prüfsubstanzen und den relativen Verlängerungen an der Aconitin-Infusionsdauer (Δ %) bestehen lineare Beziehungen aus denen als ED 50 % die Dosen bestimmt werden können, welche die Infusionsdauer um 50 % verlängern.

Zur weiteren Charakterisierung der Substanzen wird auf der Basis der in den Experimenten verwendeten dezimalgeometrischen Dosenfolge (Faktor

$$\sqrt[3]{10})$$

die Dosis ermittelt, bei welcher erste cardiotoxische Symptome (Veränderungen des Aausgangs-EKG) bzw. neurotoxische Symptome (Koordinationsstörungen, Konvulsionen usw.) auftreten. Der Quotient aus den toxischen Dosen und den antiarrhythmisch wirksamen Dosen (ED 50 %) ist eine Maßzahl für die therapeutische Breite der neuen Verbindungen.

Als Vergleichssubstanz dient das bekannte Antiarrhythmicum Chinidin.

Die erfindungsgemäßen Verbindungen (Tab. 1) sind an der Aconitinarrhythmie (Beispiel 4) 3- bis 59mal (Beispiel 39) wirksamer als Chinidin. Darüber hinaus besitzen sie eine größere therapeutische Breite als Chinidin. Die toxischen Dosen sind 12mal (Beispiel 36) bis 17mal (Beispiel 22) größer als die antiarrhythmisch wirksamen, die von Chinidin nur 11mal.

**Tabelle 1**: Antiarrhythmische Wirkung und Toxizität

Ratte, Appl.: per os

| | Aconitinarrhythmie | | Toxizität | |
|---|---|---|---|---|
| Substanz des Beisp. Nr. | ED 50 % 1) mg/kg | R.W. | Dosis 3) mg/kg | Q 4) |
| 4 | 13,5 | 3,22 | 215 | 16 |
| 22 | 12,4 | 3,51 | 215 | 17 |
| 25 | 1,71 | 25,44 | 21,5 | 13 |
| 27 | 1,57 | 27,71 | 21,5 | 14 |
| 36 | 0,842 | 51,66 | 10 | 12 |
| 37 | 0,803 | 54,17 | 10 | 13 |
| 39 | 0,732 | 59,43 | 4,64 | 14 |
| Chinidin | 43,5 | 1,00 | 215 | 11 |

1) Dosis (mg/kg), welche die Aconitininfusionsdauer um 50 % verlängert.
2) R.W. = Relative Wirksamkeit, bezogen auf Chinidin = 1,00
3) Dosis (mg/kg) nach deren Applikation die ersten toxischen Symptome beobachtet werden
4) $Q = \frac{\text{Toxische Dosis (mg/kg)}}{\text{ED 50 \% (mg/kg)}}$

Gegenstand der vorliegenden Erfindung sind daher weiter Arzneimittel, die eine Verbindung der Formel I oder deren physiologisch verträgliches Säureadditionssalz enthalten, sowie die Verwendung der neuen Verbindungen bei der Behandlung von Krankheiten.

Die neuen Verbindungen können in den üblichen galenischen Applikationsformen, fest oder flüssig, angewendet werden, wie Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die Dosierung der erfindungsgemäßen Verbindungen hängt vom Alter, Zustand und Gewicht des Patienten sowie von der Applikationsart ab. In der Regel beträgt die tägliche Wirkstoffdosis 5 bis 75 mg/kg bei oraler und 1 bis 10 mg/kg bei parenteraler Gabe.

Die vorliegende Erfindung wird durch die nachfolgenden Ausführungsbeispiele näher erläutert.

**Beispiel 1**

1-(3'-Dimethylaminopropyl)-2-acetyl-9-methyl-1,2,3,4-tetrahydro-β-carbolin

a) Herstellung des Ausgangsproduktes 1-(3'-Chlorpropyl)-2-acetyl-1,2,3,4-tetrahydro-β-carbolin

17,1 g (60 mM) 1-(3'-Chlorpropyl)-1,2,3,4-tetrahydro-β-carbolin-hydrochlorid wurden in 240 ml Dimethylformamid (DMF) unter Erhitzen gelöst und anschließend im Eisbad auf 5°C gekühlt. Unter gutem Rühren tropfte man 11,5 g (150 mM) Acetylchlorid und gleich danach (300 mM) Pyridin hinzu, wobei die Temperatur 5°C nicht überstieg. Anschließend ließ man bei derselben Temperatur 2 h nachrühren und schüttete dann das Reaktionsgemisch auf Eiswasser. Der Niederschlag wurde abgesaugt und mit Wasser gut ausgewaschen. Man isolierte 16,9 g (97 %) Kristalle, Schmp. = 166 bis 168°C (Ethanol).

1-(3'-Chlorpropyl)-2-acetyl-9-methyl-1,2,3,4-tetrahydro-β-carbolin

16,8 g (55 mM) 1-(3'-Chlorpropyl)-2-acetyl-1,2,3,4-tetrahydro-β-carbolin wurden in 100 ml DMF gelöst, mit 7,03 g (55 mM) Dimethylsulfat versetzt. Anschließend wurde eine Mischung aus 15 ml DMF und 10,4 g (55 mM) 30 %iger Natriummethylat-Lösung unter Kühlung im Eisbad zugetropft. Man ließ 1 bis 2 h bei 5°C nachrühren und goß anschließend den Ansatz unter Rühren auf 3 l Eiswasser. Der Niederschlag wurde abgesaugt, der Rückstand sofort in Methylenchlorid gelöst, getrocknet und eingeengt. Der Rückstand wurde durch Säulenchromatographie (Kieselgel, Methylenchlorid/ Methanol 95/5) oder durch Umkristallisieren aus Ethanol unter Zusatz von Aktivkohle gereinigt. Man erhielt 12,6 g (75 %), Schmp. 93 bis 95°C.

b) Herstellung des Endproduktes 1-(3'-Dimethylaminopropyl)-2-acetyl-9-methyl-1,2,3,4-tetrahydro-β-carbolin

5,8 g (18 mM) 1-(3'-Chlorpropyl)-2-acetyl-9-methyl-1,2,3,4-tetrahydro-β-carbolin wurden in 150 ml 40 %iger Dimethylamin-Lösung in Wasser suspendiert, in einen Rührautoklaven überführt und 5 h bei 150°C und Eigendruck gerührt. Anschließend engte man den Ansatz im Vakuum zur Trockene ein, löste das helle Öl in Methylenchlorid, versetzte mit Wasser, stellte die wäßrige Phase mit verdünnter Natronlauge auf pH = 10 und extrahierte die wäßrige Phase noch zweimal mit Methylenchlorid. Trocknen und Einengen der organischen Phase lieferte 5,8 g Rohprodukt, das durch Säulenchromatographie (Kieselgel, Laufmittel Methylenchlorid/Methanol 95/5) oder durch Umkristallisieren aus Ethanol unter Verwendung von Aktivkohle gereinigt wurde. Man isolierte 4,7 g (83 %) Produkt als Öl, das zur Überführung in das Hydrochlorid in Ether gelöst und nach Filtrieren mit etherischer HCl-Lösung versetzt wurde. Schmp. (Hydrochlorid) = 108 bis 110°C.

Analog Beispiel 1 wurden hergestellt:

2. 1-(3'-Methylaminopropyl)-2-acetyl-9-methyl-1,2,3,4-tetrahydro-β-carbolin, Schmp. = 63 bis 65°C.
3. 1-(3'-Diethylaminopropyl)-2-acetyl-1,2,3,4-tetrahydro-β-carbolin . 0,5 $H_2O$, Schmp. = 49 bis 50°C.
4. 1-(3'-Dimethylaminopropyl)-2-acetyl-1,2,3,4-tetrahydro-β-carbolin . 0,5 $H_2O$, Schmp. = 148 bis 150°C.
5. 1-(3'-Methylaminopropyl)-2-acetyl-1,2,3,4-tetrahydro-β-carbolin . 0,5 $H_2O$ Schmp. = 75 bis 77°C.
6. 2-(3'-Dimethylamimopropyl)-2-acetyl-6-chlor-1 2 3 4-tetrahydro-β-carbolin . $H_2O$ Schmp. = 65 bis 67°C.
7. 1-(3'-Methylaminopropyl)-2-acetyl-6-chlor-1,2,3,4-tetrahydro-β-carbolin . $H_2O$, Schmp. = 86 bis 88°C.
8. 1-(3'-Dimethylaminopropyl)-2-acetyl-9-ethyl-1,2,3,4-tetrahydro-β-carbolin . $H_2O$, Schmp. = 78 bis 80°C.
9. 1-(4'-Dimethylaminobutyl)-2-acetyl-1,2,3.4-tetrahydro-β-carbolin . $H_2O$, Schmp. = 83 bis 85°C.
10. 1-(3'-Dimethylaminopropyl)-2-acetyl-6-chlor-9-methyl -1,2,3,4-tetrahydro-β-carbolin, Schmp. = 49 bis 50°C.
11. 1-(4'-Dimethylaminobutyl)-2-acetyl-9-methyl-1,2,3,4-tetrahydro-β-carbolin . 1,5 $H_2O$, Schmp. = 44 bis 45°C.

Analog Beispiel 1b wurden unter Erhöhung der Reaktionstemperatur auf 155 bis 180°C und der Reaktionszeit auf 6 bis 12 h und unter Verwendung des jeweiligen reinen Amins hergestellt:

12. 1-(3'-Diisopropylaminopropyl)-2-acetyl-9-methyl-1,2,3,4-tetrahydro-β-carbolin . 0,5 HCl, Schmp. = 56 bis 58°C.
13. 1-(3'-Diisopropylaminopropyl)-2-acetyl-1,2,3,4-tetrahydro-β-carbolin . 0,5 $H_2O$ Schmp. = 161 bis 162°C.
14. 1-[3'-(2-Butyl)-isopropylaminopropyl]-2-acetyl-9-methyl -1,2,3,4-tetrahydro-β-carbolin . HCl . 2 $H_2O$, Schmp. = 98 bis 99°C.

15. 1-(4'-Diisopropylaminobutyl)-2-acetyl-1,2,3,4-tetrahydro-β-carbolin . 0,5 $H_2O$ Schmp. = 58 bis 59° C.
16. 1-(3'-Diisopropylaminopropyl)-2-acetyl-6-chlor-9-methyl -1,2,3,4-tetrahydro-β-carbolin, Schmp. = 50 bis 52° C.
17. 1-[3'-(Di-2-butyl)-aminopropyl]-2-acetyl-9-methyl-1,2,3,4-tetrahydro-β-carbolin, Öl.
18. 1-[3'-(t-Butyl)-aminopropyl]-2-acetyl-9-methyl-1,2,3,4-tetrahydro-β-carbolin, Öl.
19. 1-[3'-(t-Butyl)-aminopropyl]-2-acetyl-1,2,3,4-tetrahydro-β-carbolin, Schmp. = 69 bis 71° C.

**Beispiel 20** (Herstellungsbeispiel)

1-[3'-(4-Methyl-piperazin-1-yl)-propyl]-2-acetyl -1,2,3,4-tetrahydro-β-car-bolin . $H_2O$

6,5 g (22 mM) 1-(3'-Chlorpropyl)-2-acetyl-1,2,3,4-tetrahydro-β-carbolin wurden mit 10,0 g (100 mM) N-Methylpiperazin versetzt und 2 h bei 80° C gerührt. Nach dem Abkühlen destillierte man das überschüssige Amin im Vakuum ab, nahm den Ansatz in Eiswasser auf, extrahierte mit Methylenchlorid, wusch mit $H_2O$ grundlich aus, trocknete die organische Phase und engte ein. Das Rohprodukt wurde anschließend durch Säulenchromatographie (Kieselgel, Laufmittel Methylenchlorid/Methanol 95/5) oder durch Umkristallisieren aus Ethanol unter Zusatz von Aktivkohle gereinigt. Man isolierte 6,1 g (74 %), Schmp. = 165 bis 167° C.

Analog Beispiel 20 wurden bei einer Reaktionstemperatur von 120 bis 180° C und einer Reaktionszeit von 3 bis 24 h hergestellt:

21. 1-(3'-Isopropylaminopropyl)-2-acetyl-1,2,3,4-tetrahydro-β-carbolin . $H_2O$, Schmp. = 100 bis 102° C.
22. 1-(3'-n-Butylaminopropyl)-2-acetyl-1,2,3,4-tetrahydro-β-carbolin . 0,5 $H_2O$, Öl.
23. 1-[3'-(2-Butyl)-aminopropyl]-2-acetyl-1,2,3,4-tetrahydro-β-carbolin . 0,5 $H_2O$, Öl.
24. 1-[3'-(2-Methylpropyl)-aminopropyl]-2-acetyl -1,2,3,4-tetrahydro-β-carbolin . 0,5 $H_2O$, Öl.
25. 1-[3'-(Di-n-butyl)-amimopropyl]-2-acetyl-1,2,3,4-tetrahydro-β-carbolin . 0,5 $H_2O$ Schmp. = 81 bis 83° C.
26. 1-[3'-(Di-2-methylpropyl)-aminopropyl]-2-acetyl-1,2,3,4-tetrahydro-β-carbolin, Schmp. = 138 bis 140° C.
27. 1-[3'-(Di-2-butyl)-aminopropyl]-2-acetyl-1,2,3,4-tetrahydro-β-carbolin . 0,5 HCl, Schmp. = 139 bis 141° C.
28. 1-(3'-Propylaminopropyl)-2-acetyl-1,2,3,4-tetrahydro-β-carbolin . $H_2O$, Öl.
29. 1-[3'-(Di-propyl)-aminopropyl]-2-acetyl-1,2,3,4-tetrahydro-β-carbolin, Schmp. = 106 bis 108° C.
30. 2-(3'-Propylaminopropyl)-2-acetyl-9-methyl-1,2,3,4-tetrahydro-β-carbolin . $H_2O$, Öl.
31. 1-(3'-Isopropylaminopropyl)-2-acetyl-9-methyl-1,2,3,4-tetrahydro-β-carbolin . $H_2O$, Öl.
32. 1-(3'-n-Butylaminopropyl)-2-acetyl-9-methyl-1,2,3,4-tetrahydro-β-carbolin . HCl . $H_2O$, Schmp. = 79 bis 81° C.
33. 1-[3'-(2-Butyl)-aminopropyl]-2-acetyl-9-methyl-1,2,3,4-tetrahydro-β-carbolin, Öl.
34. 1-(3'-Diethylaminopropyl)-2-acetyl-9-methyl-1,2,3,4-tetrahydro-β-carbolin . 0,5 $H_2O$, Öl.
35. 1-[3'-(Di-n-butyl-aminopropyl]-2-acetyl-9-methyl-1,2,3,4-tetrahydro-β -carbolin, Öl.
36. 1-(3'-Dipropylaminopropyl)-2-acetyl-9-methyl-1,2,3,4-tetrahydro-β-carbolin . HCl . 1,5 $H_2O$, Schmp. = 87 bis 89° C.
37. 1-[3'-(Di-2-methylpropyl)-aminopropyl]-2-acetyl-9-methyl-1,2,3,4-tetrahydro-β-carbolin, Schmp. = 137 bis 139° C, Schmp. (Hydrochlorid . $H_2O$) = 87 bis 89° C.
38. 1-(3'-Methyl-isopropylaminopropyl)-2-acetyl-9-methyl -1,2,3,4-tetrahydro-β-carbolin . HCl . 2 $H_2O$ Schmp. = 81 bis 83° C.
39. 1-[3'-(Isopropyl-propyl)-aminopropyl]-2-acetyl-9-methyl-1,2,3,4-tetrahydro-β-carbolin . HCl . 2 $H_2O$, Schmp. = 91 bis 93° C.
40. 1-[3'-(n-Butyl)-isopropylaminopropyl]-2-acetyl-9-methyl-1,2,3,4-tetrahydro-β-carbolin . HCl . $H_2O$, Schmp. = 106 bis 108° C.
41. 1-[3'-(2-Methylpropyl)-isopropylaminopropyl]-2-acetyl-9-methyl-1,2,3,4-tetrahydro-β-carbolin . HCl . 2 $H_2O$, Schmp. = 128 bis 130° C.
42. 1-[3'-(Di-2-methylpropyl)-aminopropyl]-2-acetyl-6,9-dimethyl-1,2,3,4-tetrahydro-β-carbolin, Schmp. = 108 bis 110° C.
43. 1-(3'-Dipropylaminopropyl)-2-acetyl-6,9-dimethyl-1,2,3,4-tetrahydro-β-carbolin, Öl.
44. 1-[3'-(Di-2-methylpropyl)-aminopropyl]-2-acetyl-6-chlor-9-methyl-1,2,3,4-tetrahydro-β-carbolin, Öl.
45. 1-(3'-Dipropylaminopropyl)-2-acetyl-6-chlor-9-methyl-1,2,3,4-tetrahydro-β-carbolin, Öl.
46. 1-(4'-Dipropylaminobutyl)-2-acetyl-9-methyl-1,2,3,4-tetrahydro-β-carbolin . $H_2O$, Öl.
47. 1-[4'-(Di-2-methylpropyl)-aminobutyl]-2-acetyl-9-methyl-1,2,3,4-tetrahydro-β-carbolin . 0,5 $H_2O$, Schmp. = 86 bis 88° C.
48. 1-(4'-Dipropylaminobutyl)-2-acetyl-1,2,3,4-tetrahydro-β-carbolin . 0,5 $H_2O$, Öl.
49. 1-[4'-(Di-2-methylpropyl)-aminobutyl]-2-acetyl-1,2,3,4-tetrahydro-β-carbolin . 0,5 $H_2O$, Schmp. = 126 bis 128° C.
50. 1-[3'-(2,6-Dimethyl-piperidin-1-yl)-propyl]-2-acetyl-9-methyl-1,2,3,4-tetrahydro-β-carbolin . 0,5 $H_2O$, Schmp. = 153 bis 155° C.

**Beispiel 51**

1-[3'-(4-Methyl-piperazin-1-yl)-propyl]-2-acetyl-6-chlor-1,2,3,4-tetrahydro-β-carbolin . 0,5 HCl

5,0 g (16 mM) 1-(3'-Chlorpropyl)-2-acetyl-6-chlor-1,2,3,4-tetrahydro-β-carbolin in 30 ml Dimethylformamid wurden mit 1,6 g (16 mM) N-Methylpiperazin und anschließend mit 1,7 g (16 mM) Triethylamin versetzt. Man ließ das Gemisch 3 h bei 120°C rühren und goß den Ansatz nach dem Abkühlen auf Eiswasser, extrahierte mehrmals mit Methylenchlorid, wusch die organische Phase gut mit Wasser aus, trocknete und engte ein. Das Rohprodukt wurde durch Säulenchromatographie (Kieselgel, Laufmittel Methylenchlorid/Methanol 95/5) oder durch Umkristallisieren aus Ethanol unter Zusatz von Aktivkohle gereinigt. Man isolierte 4,1 g (63 %), Zersetzungspunkt = 80°C.

Analog Beispiel 51 wurden hergestellt:

52. 1-(3'-Piperidin-1-yl-Propyl)-2-acetyl-1,2,3,4-tetrahydro-β-carbolin . 0,5 HCl, Schmp. = 134 bis 136°C.

Analog den Beispielen 1, 20 und 51 wurden hergestellt:

53. 1-(3'-Dipropylaminopropyl)-2-propionyl-9-methyl-1,2,3,4-tetrahydro-β -carbolin, Schmp. 72-74°C.
54. 1-[3'-(Di-2-methylpropyl)-aminopropyl]-2-propionyl-9-methyl-1,2,3,4 -tetrahydro-β-carbolin, Schmp. 110-112°C.
55. 1-(3'-Dipropylaminopropyl)-2-propionyl-1,2,3,4-tetrahydro-β-carbolin, Öl.
56. 1-[3'-(Di-2-methylpropyl)-aminopropyl]-2-propionyl-1,2,3,4-tetrahydro-β-carbolin, Schmp. 135-137°C.
57. 1-[3'-(n-Butyl-2-methylpropyl)-aminopropyl]-2-acetyl-9-methyl-1,2,3,4-tetrahydro-β-carbolin, Schmp. 102-104°C.
58. 1-[3'-(n-Butyl-isopropyl)-aminopropyl]-2-acetyl-9-methyl-1,2,3,4-tetrahydro-β-carbolin, Öl.
59. 1-[3-(2-Methylpropyl-propyl)-aminopropyl]-2-acetyl-9-methyl-1,2,3,4 -tetrahydro-β-carbolin, Schmp. 100-101°C.
60. 1-[3'-(2-Methylpropyl-ethyl)-aminopropyl]-2-acetyl-9-methyl-1,2,3,4-tetrahydro-β-carbolin, Schmp. 64-65°C.

Analog den Beispielen 1, 20 und 51 lassen sich herstellen.

61. 1-[3'-(Di-n-butyl)-aminopropyl]-2-propionyl-9-methyl-1,2,3,4-tetra-hydro-β-carbolin.
62. 1-[3'-(Di-2-butyl)-aminopropyl]-2-propionyl-1,2,3,4-tetrahydro-β-carbolin.
63. 1-[3'-(Di-n-butyl)-aminopropyl]-2-propionyl-1,2,3,4-tetrahydro-β-carbolin.
64. 1-(3'-Dipropylaminopropyl)-2-acetyl-6-methoxy-1,2,3,4-tetrahydro-β-carbolin.
65. 1-[3'-(Di-2-methylpropyl)-aminopropyl]-2-acetyl-6-methoxy-1,2,3,4-tetrahydro-β-carbolin.

**Galenisches Beispiel**

Auf einer Tablettenpresse werden in üblicher Weise Tabletten folgender Zusammensetzung gepreßt:

100 mg Wirkstoff des Beispiels 37
150 mg Lactose
30 mg Maisstärke
36 mg Cellulose
10 mg Polyvinylpyrrolidon
4 mg Magnesiumstearat.

**Patentansprüche** fü die Vertragsstaaten BE, CH, DE, FR, GB, IT, LI, NL, SE.

1. 2-Substituierte 1(Aminoalkyl)-1,2,3,4-tetrahydro-β-carboline der Formel I

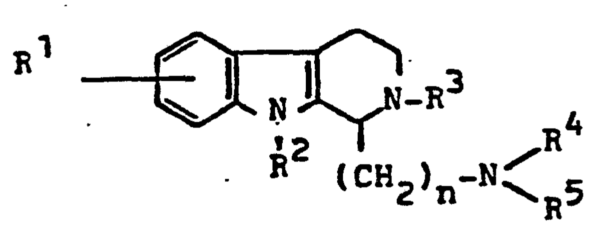

I,

in der
n eine ganze Zahl von 2 bis 5,
$R^1$ ein Wasserstoff- oder Halogenatom oder einen Trifluormethyl-, $C_1$-$C_4$-Alkyl- oder $C_1$-$C_4$-Alkoxyrest,
$R^2$ ein Wasserstoffatom oder einen $C_1$-$C_3$-Alkylrest,
$R^3$ einen $C_1$-$C_3$-Acylrest,
$R^4$ einen Alkylrest mit bis zu 4 C-Atomen und
$R^5$ ein Wasserstoffatom oder einen Alkylrest mit bis zu 4 C-Atomen und
$R^4$ und $R^5$ zusammen mit dem N-Atom auch einen Piperidinring, der durch eine oder mehrere $C_{1-3}$-Alkylgruppen substituiert sein kann, bedeuten, sowie deren Salze mit physiologisch verträglichen Säuren.

2. 1-[3'-(Di-2-methylpropyl)-aminopropyl]-2-acetyl-9-methyl-1,2,3,4-tetrahydro-β-carbolin.
3. 1-(3'-Dipropylaminopropyl)-2-acetyl-9-methyl-1,2,3,4-tetrahydro-β-carbolin.
4. 1-(3'-Isopropyl-propylaminopropyl)-2-acetyl-9-methyl-1,2,3,4-tetrahydro-β-carbolin
5. 1-[3'-(Di-2-butyl)-aminopropyl]-2-acetyl-1,2,3,4-tetrahydro-β-carbolin.
6. 1-[3'-(Di-n-butyl)-aminopropyl]-2-acetyl-1,2,3,4-tetrahydro-β-carbolin.
7. Arzneimittel, enthaltend eine Verbindung der Formel I, gemäß Anspruch 1.
8. Verbindung der Formel I gemäß Anspruch 1 zur Verwendung bei der Bekämpfung von Krankheiten.
9. Verfahren zur Herstellung der Verbindungen der Formel I gemäß Anspruch 1, dadurch gekennzeichnet, daß man eine Verbindung der Formel II

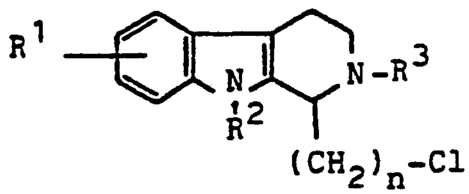

II

in der n, $R^1$, $R^2$ und $R^3$ die angegebenen Bedeutungen haben, mit einem Amin der Formel
$HNR^4R^5$,
worin $R^4$ und $R^5$ die angegebenen Bedeutungen haben, umsetzt und die so erhaltenen Verbindungen gegebenenfalls in ihre Salze mit physiologisch verträglichen Säuren überführt.

**Patentansprüche** für den Vertragsstaat: AT.

1. Verfahren zur Herstellung 2-substituierter 1-(-Aminoalkyl)-1,2,3,4-tetrahydro-β-carboline der Formel I

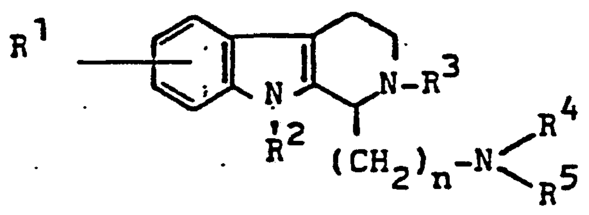

I,

in der
n eine ganze Zahl von 2 bis 5,
$R^1$ ein Wasserstoff- oder Halogenatom oder einen Trifluormethyl-, $C_1$-$C_4$-Alkyl- oder $C_1$-$C_4$-Alkoxyrest,
$R^2$ ein Wasserstoffatom oder einen $C_1$-$C_3$-Alkylrest,
$R^3$ einen $C_1$-$C_3$-Acylrest,
$R^4$ einen Alkylrest mit bis zu 4 C-Atomen und
$R^5$ ein Wasserstoffatom oder einen Alkylrest mit bis zu 4 C-Atomen und
$R^4$ und $R^5$ zusammen mit dem N-Atom auch einen Piperidinring, der durch eine oder mehrere $C_{1-3}$-Alkylgruppen substituiert sein kann,
bedeuten, sowie deren Salze mit physiologisch verträglichen Säuren, dadurch gekennzeichnet, daß man eine Verbindung der Formel II

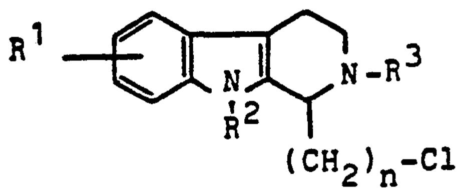

II

in der n, $R^1$, $R^2$ und $R^3$ die angegebenen Bedeutungen haben, mit einem Amin der Formel $HNR^4R^5$,
worin $R^4$ und $R^5$ die angegebenen Bedeutungen haben, umsetzt und die so erhaltenen Verbindungen gegebenenfalls in ihre Salze mit physiologisch verträglichen Säuren überführt.

2. Verfahren gemäß Anspruch 1, dadurch gekennzeichnet, daß man 1-[3'-(Di-2-methylpropyl)-aminopropyl]-2-acetyl-9-methyl-1,2,3,4-tetrahydro-β-carbolin herstellt.

3. Verfahren gemäß Anspruch 1, dadurch gekennzeichnet, daß man 1-(3'-Dipropylaminopropyl)-2-acetyl-9-methyl-1,2,3,4-tetrahydro-β-carbolin herstellt.

4. Verfahren gemäß Anspruch 1, dadurch gekennzeichnet, daß man 1-(3'-Isopropyl-propylaminopropyl)-2-acetyl-9-methyl-1,2,3,4-tetrahydro-β-carbolin herstellt.

5. Verfahren gemäß Anspruch 1, dadurch gekennzeichnet, daß man 1-[3'-(Di-2-butyl)-aminopropyl]-2-acetyl-1,2,3,4-tetrahydro-β-carbolin herstellt.

6. Verfahren gemäß Anspruch 1, dadurch gekennzeichnet, daß man 1-[3'-(Di-n-butyl)-aminopropyl]-2-acetyl-1,2,3,4-tetrahydro-β-carbolin herstellt.

**Claims** for the Contracting States: BE, CH, DE, FR, GB, IT, LI, NL, SE.

1. A 2-substituted 1-(aminoalkyl)-1,2,3,4-tetrahydro-β-carboline of the formula

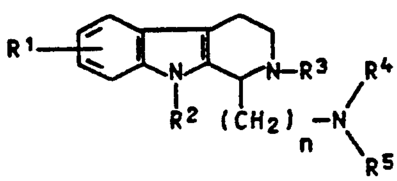

I,

where
n is an integer from 2 to 5,
$R^1$ is hydrogen halogen, trifluoromethyl, $C_1$-$C_4$-alkyl or $C_1$-$C_4$-alkoxy,
$R^2$ is hydrogen or $C_1$-$C_3$-alkyl
$R^3$ is $C_1$-$C_3$-acyl,
$R^4$ is alkyl of up to 4 carbon atoms and
$R^5$ is hydrogen or alkyl of up to 4 carbon atoms, or
$R^4$ and $R^5$ together with the nitrogen atom form a piperidine ring which can be substituted by one or more $C_1$-$C_3$-alkyl groups, and its salts with physiologically tolerated acids.

2. 1-[3'-(Di-2-methylpropyl)-aminopropyl]-2-acetyl-9-methyl-1,2,3,4-tetrahydro-β-carboline.

3. 1-(3'-Dipropylaminopropyl)-2-acetyl-9-methyl-1,2,3,4-tetrahydro-β-carboline.

4. 1-(3 -Isopropylpropylaminopropyl)-2-acetyl-9-methyl-1,2,3 4-tetrahydro-β-carboline.

5. 1-[3'-(Di-2-butyl)-aminopropyl]-2-acetyl-1 2,3 4-tetrahydro-β-carboline.

6. 1-[3'-(Di-n-butyl)-aminopropyl]-2-acetyl-1,2,3 4-tetrahydro-β-carboline.

7. A pharmaceutical containing a compound of the formula I as claimed in claim 1.

8. A compound of the formula I as claimed in claim 1 for use in the treatment of disorders.

9. A process for the preparation of a compound of the formula I as claimed in claim 1, wherein a compound of the formula II

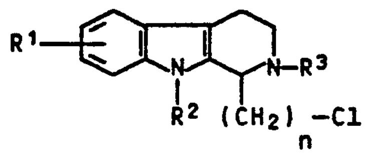

II.

where n, $R^1$, $R^2$ and $R^3$ have the specified meanings, is reacted with an amine of the formula $HNR^4R^5$,

where $R^4$ and $R^5$ have the specified meanings, and, if desired, the compound thus obtained is converted into its salts with physiologically tolerated acids.

**Claims** for the Contracting State: AT.

1. A process for the preparation of a 2-substituted 1-(aminoalkyl)-1,2,3,4-tetrahydro-β-carboline of the formula 1

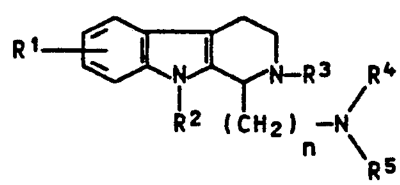

I.

where

n is an integer from 2 to 5,

$R^1$ is hydrogen, halogen, trifluoromethyl, $C_1$-$C_4$-alkyl or $C_1$-$C_4$-alkoxy,

$R^2$ is hydrogen or $C_1$-$C_3$-alkyl

$R^3$ is $C_1$-$C_3$-acyl

$R^4$ is alkyl of up to 4 carbon atoms, and

$R^5$ is hydrogen or alkyl of up to 4 carbon atoms or

$R^4$ and $R^5$, together with the nitrogen atom form a piperidine ring which can be substituted by one or more $C_1$-$C_3$-alkyl groups, or its salts with physiologically tolerated acids, wherein a compound of the formula II

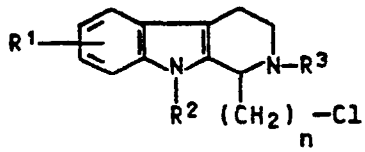

II,

where n, $R^1$, $R^2$ and $R^3$ have the specified meanings, is reacted with an amine of the formula
$HNR^4R^5$,
where $R^4$ and $R^5$ have the specified meanings, and, if desired, the compound thus obtained is converted into its salts with physiologically tolerated acids.

2. A process as claimed in claim 1, wherein 1-[3'-(di-2-methylpropyl)-aminopropyl]-2-acetyl-9-methyl-1 2,3,4-tetrahydro-β-carboline is prepared.

3. A process as claimed in claim 1, wherein 1-(3'-dipropylamino-propyl)-2-acetyl-9-methyl-1 2,3 4-tetrahydro-β-carboline is prepared.

4. A process as claimed in claim 1, wherein 1-(3'-isopropylpropylamino-propyl)-2-acetyl-9-methyl-1,2,3,4-tetrahydro-β-carboline is prepared.

5. A process as claimed in claim 1, wherein 1-[3'-(di-2-butyl)-amino-propyl]-2-acetyl-1 2,3,4-tetrahydro-β-carboline is prepared.

6. A process as claimed in claim 1, wherein 1-[3'-(di-n-butyl)-amino-propyl]-2-acetyl-1,2,3,4-tetrahydro-β-carboline is prepared.

**Revendications** pour les Etats contractants: BE, CH, DE, FR, GB, IT, LI,

NL, SE.

1. 1(aminoalkyle)-1,2,3,4-tétrahydro-β-carbolines substituées en 2, de formule I

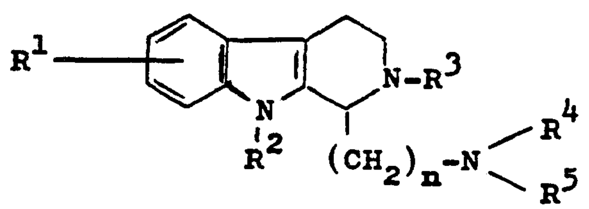

I,

dans laquelle
n est un nombre entier de 2 à 5
$R^1$ represente un atome d'hydrogène ou d'halogène ou un reste trifluorométhyle, alkyle en $C_1$-$C_4$ ou alcoxy en $C_1$-$C_4$,
$R^2$ un atome d'hydrogène ou un reste alkyle en $C_1$-$C_3$
$R^3$ un reste acyle en $C_1$-$C_3$
$R^4$ un reste alkyle ayant jusqu'à 4 atomes C, et
$R^5$ un atome d'hydrogène ou un reste alkyle ayant jusqu'à 4 atomes C et
$R^4$ et $R^5$ ensemble avec l'atome N, aussi un cycle pipéridine qui peut être substitué par un ou plusieurs groupes alkyle en $C_{1-3}$,
ainsi que leurs sels d'acides physiologiquement tolérables.

2. 1 -[3'-(di-2-méthylpropyl)-aminopropyl]-2-acétyl-9-méthyl-1,2,3,4-tétrahydro-β-carboline.

3. 1- (3'-dipropylaminopropyl)-2-acétyl-9-méthyl-1,2,3,4-tétrahydro-β-carboline.
4. 1-(3,-isopropyl-propylaminopropyl)-2-acétyl-9-méthyl-1,2,3,4-tétrahydro-β-carboline.
5. 1-[3'-(di-2-butyl)-aminopropyl]-2-acétyl-1,2,3,4-tétrahydro-β-carboline.
6. 1-[3'-(di-n-butyl)-amlnopropyl]-2-acétyl-1,2,3,4-tétrahydro-β-carboline.
7. Médicament contenant un composé de formule I selon la revendication 1.
8. Composé de formule I selon la revendication 1, pour utilisation dans la lutte contre les maladies.
9. Procédé de préparation de composés de formule I selon la revendication 1, caractérisé par le fait que l'on fait réagir un composé de formule II

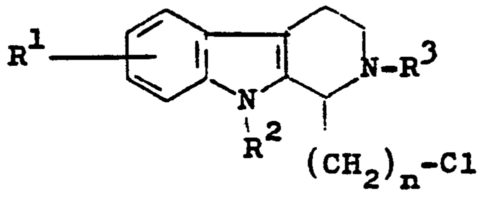

II

dans laquelle n, $R_1$, $R_2$ et $R_3$ ont les significations indiquées, avec une amine de formule
$HNR^4R^5$,
où $R^4$ et $R^5$ ont les significations indiquées et on transforme éventuellement les composés ainsi obtenus en sels d'acide physiologiquement tolerables.

**Revendications** pourl'Etat contractant: AT.

1. Procédé de préparation de 1(aminoalkyle)-1,2,3,4-tétrahydro-β-carbolines substituées en 2, de formule I

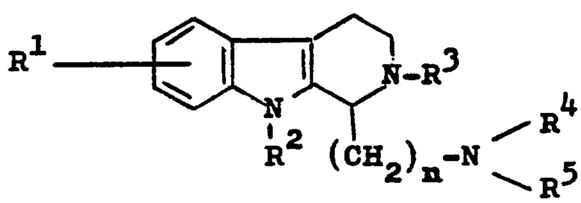

I,

dans laquelle
n est un nombre entier de 2 à 5
$R^1$ représente un atome d'hydrogène ou d'halogène ou un reste trifluorométhyle, alkyle en $C_1$-$C_4$ ou alcoxy en $C_1$-$C_4$,
$R^2$ un atome d'hydrogène ou un reste alkyle en $C_1$-$C_3$
$R^3$ un reste acyle en $C_1$-$C_3$
$R^4$ un reste alkyle ayant jusqu'à 4 atomes C, et
$R^5$ un atome d'hydrogène ou un reste alkyle ayant jusqu'à 4 atomes C et
$R^4$ et $R^5$, ensemble avec l'atome N, aussi un cycle pipéridine qui peut être substitué par un ou plusieurs groupes alkyle en $C_{1-3}$,
ainsi que leurs sels d'acides physiologiquement tolérables, caractérisé par le fait que l'on fait réagir un composé de formule II

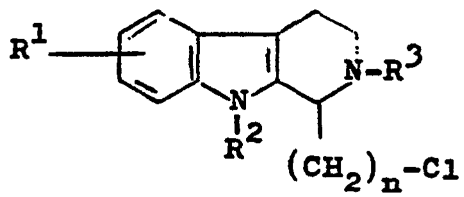

II

dans laquelle n, $R^1$, $R^2$ et $R^3$ ont les significations indiquées, avec une amine de formule
$HNR^4R^5$,
où $R^4$ et $R^5$ ont les significations indiquées et on transforme éventuellement les composés ainsi obtenus en sels d'acide physiologiquement tolérables.

2.- Procédé selon la revendication 1, caractérisé par le fait qu'on prépare la 1-(3'-(di-2-méthylpropyl)-aminopropyl)-2-acétyl-9-méthyl-1,2,3,4-tétrahydro-β-carboline.

3.- Procédé selon la revendication 1, caractérisé par le fait qu'on prépare la 1-(3'-dipropylaminopropyl)-2-acétyl-9-méthyl-1,2,3,4-tétrahydro-β-carboline.

4.- Procédé selon la revendication 1, caractérisé par le fait qu'on prépare la 1-(3'-isopropyl-propylaminopropyl)-2-acétyl-9-méthyl-1,2,3,4-tétrahydro-β-carboline.

5.- Procédé selon la revendication 1, caractérisé par le fait qu'on prépare la 1-[3'-(di-2-butyl)-aminopropyl]-2-acétyl-1,2,3,4-tétrahydro-β-carboline.

6.- Procédé selon la revendication 1, caractérisé par le fait qu'on prépare la 1-[3'-(di-n-butyl)-aminopropyl]-2-acétyl-1,2,3,4-tétrahydro-β-carboline.